# EUROPEAN PATENT SPECIFICATION

(11) **EP 1 727 781 B1**
(45) Date of publication and mention of the grant of the patent: **06.05.2009**
(21) Application number: 03782766.4
(22) Date of filing: 29.12.2003
(51) Int. Cl.: C07C 209/10, C07C 209/34, C07C 211/55, C07C 211/56

(54) **PROCESS FOR THE PREPARATION OF HIGH QUALITY 3,3',4,4'-TETRAMINOBIPHENYL**
VERFAHREN ZUR HERSTELLUNG VON 3,3',4,4'-TETRAAMINOBIPHENYL
PROCEDE DE PRODUCTION DE 3,3',4,4'-TETRA-AMINOBIPHENYLE DE HAUTE QUALITE

(43) Date of publication of application: 06.12.2006
(73) Proprietor: Council of Scientific and Industrial Research, New Dehli 110 067 (IN)
(72) Inventor: MANER, Asif, Pune District Maharashtra (IN); BAVIKAR, Sudhir, Pashan Road Pune 411008 Maharashtra (IN); SUDALAI, Arumugam, 411 008 Maharashtra (IN); SIVARAM, Swaminathan, 411 008 Maharashtra (IN)
(74) Representative: Curtis, Philip Anthony
(86) International application number: PCT/IN2003/000403
(87) International publication number: WO 2005/063679

(56) References cited:
- US-A- 3 943 175
- US-A- 5 041 666
- US-A- 5 235 105
- VOGEL, H. AND MARVEL, C.S.: "Polybenzimidazoles.II" JOURNAL OF POLYMER SCIENCE, PART A, vol. 1, 1963, pages 1531-1541, XP002290993
- DATABASE BEILSTEIN [Online] BEILSTEIN INSTITUTE FOR ORGANIC CHEMISTRY, FRANKFURT-MAIN, DE; XP002290994 retrieved from XFIRE accession no. RID9132004 & TAKAMURA-ENYA, T.; WATANABE, T. ET AL.: "Identification of a new mutagenic polychlorinated biphenyl derivative in the Waka river, Wakayama, Japan, showing activation of an aryl hydrocarbon receptor-dependent transcription" CHEM. RES. TOXICOL., vol. 15, no. 3, 2002, pages 419-425,
- DATABASE BEILSTEIN [Online] BEILSTEIN INSTITUTE FOR ORGANIC CHEMISTRY, FRANKFURT-MAIN, DE; XP002290995 retrieved from XFIRE accession no. RID8201608 & BORSCHE; SCHOLTEN: CHEM. BER., vol. 50, 1917, page 608,

## Description

### Field of the present invention

The present invention relates to a process for the preparation of pure high quality 3,3', 4,4'-tetraminobiphenyl (TAB) of formula 1 in high yields.

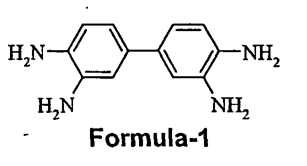

### Background and prior art references of the present Application

3,3', 4,4'-Tetraminobiphenyl (TAB) is a valuable intermediate and final product in various areas. For example, TAB is used as monomer in the preparation of polybenzimidazole (PBI) polymers, which are characterized by excellent thermal and mechanical stability. These PBI polymers are widely used as proton-conducting materials for fuel cell applications (compare U.S. Pat. Nos. 2,895,948, 3,174,947, 5,317,078 and 6,187,231). TAB is also used as an antioxidant and as an agent for stabilizing epoxide resins.

In the prior art, TAB has been prepared by three known methods. One such known method is ammonolysis of 3,3'-dichlorobenzidine (DAB) in the presence of mainly Cu catalysts (both copper salts and elemental Cu) using aqueous NH₃. For example, French Patent Specification No. 1,475,631 describes such an ammonolysis of DCB, in the presence of a CuI salt and/or of Cu₂O and CaCl₂ at an elevated temperature preferably 150 - 210°C and under an elevated inert gas pressure. The crude TAB thus obtained is purified via its.salt formation with a strong acid (yield of TAB is about 70% of theory). Subsequently, various attempts were made to obtain TAB in highly pure form and in high yields from crude TAB.as described below.

The U.S. Pat. No. 3,865,876 describes the improvement on the result of the method described in the above mentioned French Patent Specifications by using essentially only CuCl as a catalyst for the ammonolysis of DCB. The yield of TAB of theory having purity of about 75-82% is between about 85 and 87 %. This product has a Cu content of about 3 - 6% by weight. The process of U.S.Pat. No. 3,943,175 (CuCl / Cu powder can also be used as catalyst, in addition to CuCl describes the purification of TAB (converting it into its sulfate by means of sulfuric acid, isolation of the sulfate and liberation there from of TAB by means of a base). The TAB thus liberated is dissolved and reprecipitated from an aqueous solution advantageously with the addition of activated charcoal and diatomaceous earth. However, the Cu content present in TAB is about 0.6 to 0.9 % and the yield at most 45.7 % of theory, relative to DCB employed.

The German Patent (Ger. Offen. DE 3, 111, 470) discloses the purification of crude TAB (obtained by ammonolysis process) by boiling it with H₂O containing activated carbon and sodium dithionate (yield of TAB is 75.9 % with ≤ 0.0005 % Cu content). The Japanese Patent (JP 60,158,146) also describes the purification of TAB by refluxing the crude TAB with activated charcoal, aq. FeCl₃ solution and hydrazine hydrate (yield of TAB: 83.2 % containing ≥10 ppm Cu) Three more patents (U.S.Pat. No. 4, 433,168, 5,235,105 and Eur. Pat. Appl. EP 522,577) describe the purification of crude. TAB (obtained from ammonolysis of DCB with copper catalyst) by crystallizing it in water in presence of 0-5 % by weight of activated carbon and about 1- 2 % by weight of a watersoluble reducing agent. (alkali metal dithionate or alkali metal sulfite) at temperature of 100-140 °C under N₂ atmosphere (yield of TAB: 88.2 % of theory with only 10 ppm Cu). In the second method for producing TAB, which has generated substantial interest, the starting material is benzidine which is acetylated with acetic anhydride, to form N, N-diacetylbenzidine. The latter compound is then nitrated with conc. HNO₃ to form 3,3'-dinitro-N, N-diacetylbenzidine which is base hydrolysed to form 3.3'-dinitrobenzidine. This is then reduced by any of various means to form TAB [H.Vogel and C.S.Marvel, J. Poly. Sci. Part Al, 1531 (1963)]

The third method describes the production of TAB from biphenyl which comprises the following six steps: (1) acetylating the biphenyl in the presence of an appropriate Friedel -Crafts catalyst to obtain 4,4'-diacetylbiphenyl (DAcB); (2) oximating the DAcB to form DAcB dioxime; (3) subjecting the dioxime to a double Beckmann rearrangement to obtain N, N-diacetylbenzidine. (DiAcBz); (4) nitrating the DiAcBz to obtain 3,3'-dinitro-N,N '-diacetylbenzidine (DNAcBz) ; (5) removing the acetyl groups of the DNAcBz by basic hydrolysis to form 3,3'-dinitrobenzidine (DNB) and (6) reducing the nitro groups of DNB to form TAB (U.S.Pat. No. 5,041,666).

All the foregoing methods suffer from the following disadvantages associated with them:
1. They utilize benzidine as one of the raw materials, which is a known carcinogen.
2. Direct ammonolysis of DCB catalyzed by copper salts requires high temperatures (200° - 300 °C) at a pressure of 6205-6894 Kpa (900-1000 psig) of making the process more hazardous from Safety point of view. Our own experience with direct ammonolysis of DCB by following the Patented procedures is very disappointing to obtain TAB as tarry materials always accompany it.
3. In the direct ammonolysis, copper forms stable complexes probably with TAB in situ so that liberating TAB from the complex becomes tedious.
4. In the direct ammonolysis, copper forms stable complexes probably with the corresponding triaminobiphenyl and little amount of copper, so that separation of these impurities from TAB becomes tedious.
5. In the dioximation method, many of steps are involved starting from expensive biphenyl with low yield.

All the above methods make use of relatively expensive starting materials and the reaction conditions are harash to carry out. Thus, any method for producing TAB utilizing a cheaper raw material, which is both safer and easier to handle, would be very desirable.

Reference is made to the following additional prior art:
Vogel, H. and Marvel C.S., "Polybenzimadoles II", Journal of Polymer Science, Part A, Vol 1., 1963, pages 1531-1541, XP002290993 discusses a reaction where the reactants are ammonia and 5,5'- dichloro-2,4,2',4'-tetranitrobiphenyl to produce 4,6,4',6'-tetranitrobiphenyl-3,3'-diyldiamine. The citation does not disclose the synthesis of 3,3',4,4'-tetraminobiphenyl (TAB) from dichlorobenzidiene in presence of heterogenous catalyst.

US3,943,175 describes the purification of 3,3'-diaminobenzidine. It does not disclose the preparation of 3,3',4,4'-tetraminobiphenyl with heterogenous catalyst.

US5235105 US5041666 deal with process for preparation of 3,3',4,4'-tetraminobiphenyl. US5235105 describes a synthetic procedure in presence of Cucl. US5041666 describes an alternative process for the preparation of 3,3',4,4'-tetraminobiphenyl, which is a multi-step complicated process involving Friedel Craft catalysts and Beckmannn rearrangement.

Takamura-Enya, T., Watanabe, T., et al, "Identification of new mutagenic polychlorinated biphenyl derivative in the Waka river, Wakayama, Japan, showing activation of an aryl hydrocarbon receptor-dependent transcription", Chem Res Toxicol, vol 15, no. 3, 2002, pages 419 to 425, XP002290994, describes the preparation of polybenzimidazoles from 3, 3' diamonobenzidine and pthalic anhydride. For the preparation of 3, 3' diamonobenzidine from 3, 3'-dinitrobenzidine stannous chloride and hydrochloride acid are used.

Borsche, Scholten, Chem Ber, vol 50, 1917, page 608, XP002290995, describes the preparation of 3,3'-dichloro-4,4'-dinitrobiphenyl by oxidation of 3,3'-dichloro benzidine with hydrogen peroxide.

### Objects of the present invention

The main object of the present invention is to provide an improved process for the preparation of pure high quality 3,3',4,4'-tetraminobiphenyl (TAB), which avoids the drawbacks as detailed above.

Another object of the present invention is to demonstrate the use of heterogeneous Ti-superoxide as a catalyst for the oxidation of DCB to obtain 3,3'-dichloro-4,4'- dinitrobiphenyl (DCDNB). DCDNB smoothly underwent ammonolysis affording 3,3'-dichloro-4, 4'dinitrobiphenyl (DADNB), followed by the reduction of nitro groups of DADNB yielded TAB in high yields.

It is therefore an object of the present invention to provide an improved process for the synthesis of TAB, which is substantially free of one or more of the disadvantages prior processes.

Yet another object is to provide a novel process for the synthesis of TAB of greater purity than heretofore possible.

### Summary of the present invention

Present application relates to a process for the preparation of pure high quality 3,3', 4,4'-tetraminobiphenyl (TAB) in high yields.

More particularly, it relates to a process for preparation of 3,3',4,4' tetraminobiphenyl (TAB) involving a three step process (1) oxidation of 3,3',-dichloro-4,4'-diaminobiphenyl (DCB) with 50% aq. H₂0₂, (2) ammonolysis of the resulting 3,3'-dinitro 4,4'-dinitrobiphenyl (DCDNB) and (3) the reduction of 3,3'-diamino-4,4'-dinitrobiphenyl (DADNB)with stannous chloride and concentrated hydrochloric acid.

### Detailed description of the present invention

Accordingly the present invention provide improved process for the preparation of pure, high quality 3,3', 4,4'-Tetraminobiphenyl (TAB) of formula 1 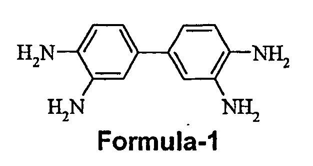 from 3, 3'- Dichloro benzidine (DCB) of formula 2 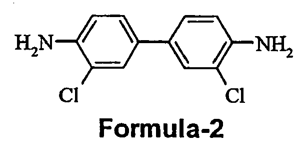 which comprises oxidizing DCB by an oxidising agent in presence of a Titanium super oxide catalyst, in presence of a solvent to obtain 3,3'dichloro 4,4'dinitro biphenyl (DCDNB) of formula (3). 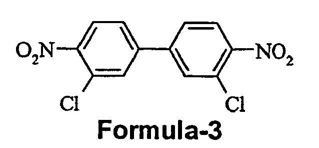 Ammonolysing DCDNB using aq. ammonia in presence of a solvent to obtain 3,3',diamino 4,4' dinitro biphenyl (DADNB) of formula (4) 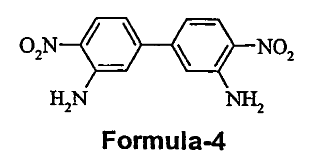 reducing DADNB using a reducing agent and basifying the resultant mixture with an alkali to obtain 3,3',4,4' tetraminobiphenyl (TAB)

Figure (2) shows reaction sequence of above described process

In one of the embodiments, the present invention the titanium super oxide used as the heterogeneous catalyst, is prepared as per procedure given in the publication [Angew. Chem. Int. Ed. Engl. 2001,40,405-408]

In another embodiment, the oxidant may be preferably H₂O₂ having the strength of 30 to 50% v/v

In yet another embodiment, the solvent used may be selected from a range of organic solvents such as but are not limited to, acetonitrile, acetone methanol, acetic acid and water.

In yet another embodiment, ammonolysis may be carried out at temperature ranging from 50 - 200 °C.

In yet another embodiment, preferred temperature for ammonolysis is 100 °C

In yet another embodiment, ammonolysis may be carried out at a pressure of 689,475-3447,3 Kpa (100-500 psig).

In yet another embodiment, ammonolysis is preferably carried out at 689,475 Kpa (100 psig.)

In yet another embodiment, reducing agents is selected from a group consisting of SnCl₂/conc. HCl mixture.

In yet another embodiment, reduction is carried out at in the temperature ranging between 50 -60 °C.

The process of the present invention is described herein with reference to examples, which are illustrative only. In order to fully illustrate the nature of the present invention and the manner of practicing the same, the following examples are presented:

### Example 1:

### Preparation of Ti-superoxide catalyst (1)

To a solution of Ti (OⁱPr)₄, (5.0 g, 0.0175 moles) in anhydrous MeOH (50mL) placed in a two-necked round bottomed flask equipped with addition funnel, nitrogen inlet and reflux condenser, 50% H₂O₂, (5.98 g, 0.175 moles) was added slowly over a period of 40 minutes under stirring at room temperature. The yellow-colored precipitate formed at once was filtered through a sintered funnel, washed with anhydrous methanol and dried under reduced pressure 399,96 Pa (3 mm Hg) at room temperature (25 °C) for 1 h, Yield: 3.94 g (98 %). EPR spectra were recorded on a Bruker EMX spectrometer operating at 9.76 GHz, 298 K and g values were determined with reference to standard marker: α, α'-diphenyl-β-picryl hydrazyl (DPPH, g = 2.0036). Thermogravimetric and differential thermal analysis (TG/DTA) was performed on TG/DTA 22, TG/DTA 32 system (Seiko Instruments Inc.) in the range of 30 - 400 °C with a temperature program 10°C /min.

### Example 2:

### Preparation of 3,3'-dichloro-4, 4'- dinitrobiphenyl (DCDNB) by oxidative procedure

In a two-necked round-bottomed flask equipped with nitrogen gas bubbler, addition funnel, water condenser and stirring magnetic bar were placed 3,3'-dichlorobenzidiene (5 g, 0.019 moles), Ti-superoxide catalyst (1), (2.5 g, 50 wt %) and anhydrous methanol (30 ml). 50 % aq. H₂O₂, (10.74 g, 0.158 moles) was added slowly under stirring for a period of 20 min. Reaction was found to be exothermic and a yellow to reddish brown color change was observed during the addition of H₂O₂. The progress of the reaction was monitored by TLC [in ethyl acetate] and after completion, catalyst 1 was filtered out and methanol evaporated under reduced pressure. The crude product weighed 4.2 g, (66.28 %) was analyzed by gas chromatography for its purity.

### Example 3:

### Preparation of 3, 3'- diamino 4, 4'- dinitrobiphenyl (DADNB) by ammonolysis procedure

A mixture of 3,3'-dichloro-4, 4'-dinitrobiphenyl (5 g, 0.0197 mol) and excess ammonia solution (100 ml), in methanol (100 ml) was charged into an autoclave and the whole mixture was heated at 100 °C for 3h. After the reaction was complete, methanol was removed by distillation and the amino product was precipitated. It was then filtered and washed with H₂O and dried. Yield 3.82 g (86.99%) m.p. 226 °C.

### Example 4:

### Preparation of 3,3'-4, 4'- tetraminobiphenyl (TAB) by reduction of nitro groups

A mixture of 3,3'-dinitrobenzidine (2 g, 0.007 mol) and stannous chloride (6.4 g, 0.034 mol) was stirred at 0 °C in ethanol (125 ml) and con. HCl (30 %) was added drop-wise over 30 min. The reaction mixture was refluxed for 10-12 h. The salt of the tetramine was precipitated out, which was basified with cold 10 % NaOH solution and the solid filtered out, washed with water dried under vacuum to give TAB in 80 % yield (1.159 g).

### The advantages of the present invention are:

1. In all three steps of reactions, the isolation of the products can be done by simple filtration.
2. The ammonolysis of DCB can be done without the use of any metal catalyst and under milder reaction temperature (100 °C) and autogenic pressure 1723,68 Kpa (250 psig).
3. The product TAB can be produced without any contamination of copper and its salts, thus enhancing the purity of TAB.
4. No by- products (diamine complexes of TAB with copper salts) are produced in the reaction mixture.
5. Quantitative conversions are obtained in all the three steps of oxidation, ammonolysis, and reduction.

## Claims

1. A process for the preparation of pure, high quality 3,3', 4,4'-Tetraminobiphenyl (TAB) of formula 1, said process comprises the 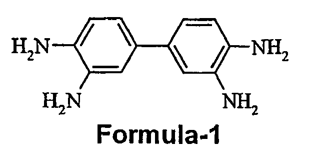 Steps of;
a. oxidizing 3,3'dichloro benzidine of formula 2 by an oxidizing agent in presence of Titanum super oxide catalyst and a solvent to obtain 3,3'dichloro4,4'dinitro biphenyl of formula3; 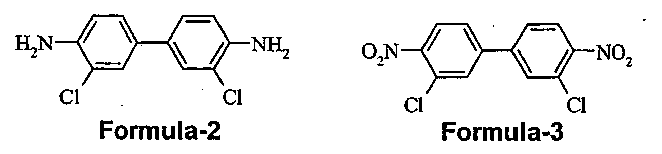
b. ammonolysing 3,3'dichloro4,4'dinitro biphenyl of formula 3 using ammonia solution in presence of a solvent to obtain 3,3',diamino 4,4' dinitro biphenyl of formula 4; 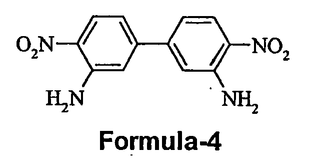
c. reducing 3,3',diamino 4,4' dinitro biphenyl of formula 4 using a reducing agent, and
d. basifying the resultant mixture with an alkali to obtain 3,3',4,4' tetraminobiphenyl of formula 1

2. A process as claimed in claim 1, wherein the present invention the titanium super oxide used as the heterogeneous catalyst.

3. A process as claimed in claim 1, wherein, the oxidant is H₂O₂ having the strength of 30 to 50% v/v.

4. A process as claimed in claim 1, wherein the solvent is organic solvent comprising of acetonitrile, acetone, methanol, acetic acid, and water.

5. A process as claimed in claim 1, wherein ammonolysis may be carried out at temperature ranging between 50 - 200 °C.

6. A process as claimed in claim 1, wherein ammonolysis is carried out at 100 °C.

7. A process as claimed in claim 1,wherein ammonolysis may be carried out at a pressure ranging between 689,475-3447,3 Kpa (100 - 500 psig.).

8. A process as claimed in claim 1,wherein ammonolysis is carried out at 689,475 Kpa (100 psig).

9. A process as claimed in claim 1,wherein reducing agents are selected from a group comprising of SnCl₂ /conc. HCl mixture

10. A process as claimed in claim 1,wherein reduction is carried out at a temperature ranging between 50 -60 °C.

## Patentansprüche

1. Verfahren zur Herstellung von reinem, hochwertigem 3,3',4,4'-Tetraaminobiphenyl (TAB) der Formel 1, wobei das Verfahren die folgenden Schritte umfasst: 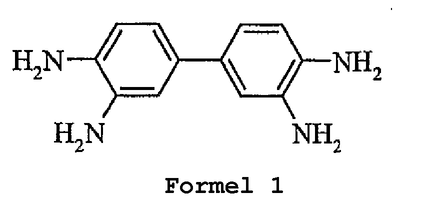
a. Oxidieren von 3,3'-Dichlorbenzidin der Formel 2 mit einem Oxidationsmittel in Gegenwart von Titanperoxid-Katalysator und einem Lösemittel, um 3,3'-Dichlor-4,4'-dinitrobiphenyl der Formel 3 zu erhalten; 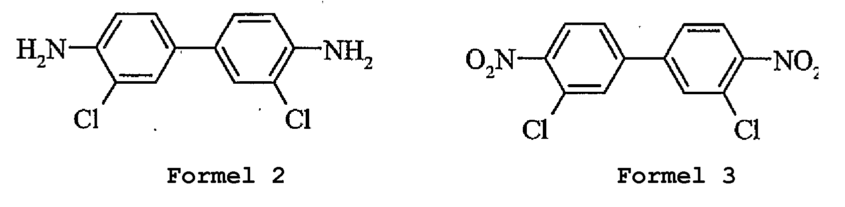
b. Ammonolysieren von 3,3'-Dichlor-4,4'-dinitrobiphenyl der Formel 3 unter Verwendung von Ammoniaklösung in Gegenwart eines Lösemittels, um 3,3'-Diamino-4,4'-dinitrobiphenyl der Formel 4 zu erhalten; 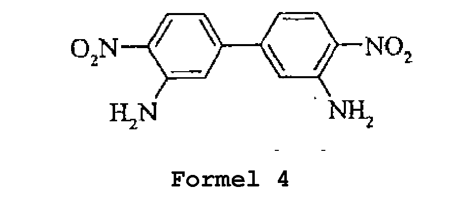
c. Reduzieren von 3,3'-Diamino-4,4'-dinitrobiphenyl der Formel 4 unter Verwendung eines Reduktionsmittels und d. Basischstellen des resultierenden Gemischs mit einem Alkali, um 3,3',4,4'-Tetraaminobiphenyl der Formel 1 zu erhalten.

2. Verfahren nach Anspruch 1, wobei in der vorliegenden Erfindung das Titanperoxid als der heterogene Katalysator verwendet wird.

3. Verfahren nach Anspruch 1, wobei es sich bei dem Oxidationsmittel um H₂O₂ mit einer Konzentration von 30 bis 50 Vol.-% handelt.

4. Verfahren nach Anspruch 1, wobei es sich bei dem Lösemittel um organisches Lösemittel handelt, das Acetonitril, Aceton, Methanol, Essigsäure und Wasser umfasst.

5. Verfahren nach Anspruch 1, wobei die Ammonolyse bei einer Temperatur durchgeführt werden kann, die zwischen 50 und 200 °C liegt.

6. Verfahren nach Anspruch 1, wobei die Ammonolyse bei 100 °C durchgeführt wird.

7. Verfahren nach Anspruch 1, wobei die Ammonolyse bei einem Druck durchgeführt werden kann, der zwischen 689,475 und 3447,3 kPa (100 und 500 psig) liegt.

8. Verfahren nach Anspruch 1, wobei die Ammonolyse bei 689,475 kPa (100 psig) durchgeführt wird.

9. Verfahren nach Anspruch 1, wobei die Reduktionsmittel aus einer Gruppe ausgewählt sind, die ein Gemisch aus SnCl₂ und konz. HCl umfasst.

10. Verfahren nach Anspruch 1, wobei die Reduktion bei einer Temperatur durchgeführt wird, die zwischen 50 und 60 °C liegt.

## Revendications

1. Procédé de préparation de 3,3', 4,4'-tétraminobiphényle (TAB) pur de haute qualité de formule 1, 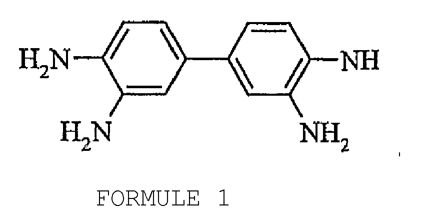 ledit procédé comprenant les étapes consistant à ;
a. oxyder de la 3,3'-dichlorobenzidine de formule 2 par un agent oxydant en présence d'un catalyste superoxyde de titane et d'un solvant pour obtenir du 3,3'-dichloro-4,4'-dinitrobiphényle de formule 3 ; 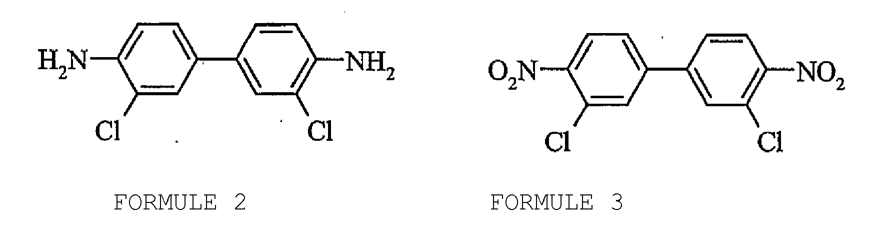
b. ammonolyser le 3,3'-dichloro-4,4'-dinitrobiphényle de formule 3 en utilisant une solution d'ammoniac en présence d'un solvant pour obtenir du 3,3'-diamino-4,4'-dinitrobiphényle de formule 4 ; 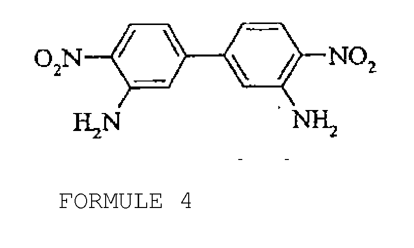
c. réduire le 3,3'-diamino-4,4'-dinitrobiphényle de formule 4 à l'aide d'un agent de réduction, et
d. alcaliniser le mélange résultant avec un alcali pour obtenir le 3,3', 4,4'-tétraminobiphényle de formule 1.

2. Procédé tel que revendiqué à la revendication 1, dans lequel le superoxyde de titane de la présente invention est utilisé comme catalyste hétérogène.

3. Procédé tel que revendiqué à la revendication 1, dans lequel l'oxydant est H₂O₂ présentant une teneur volume/volume de 30 à 50 %.

4. Procédé tel que revendiqué à la revendication 1, dans lequel le solvant est un solvant organique comprenant de l'acétonitrile, de l'acétone, du méthanol, de l'acide acétique, et de l'eau.

5. Procédé tel que revendiqué à la revendication 1, dans lequel l'ammonolyse peut être effectuée à une température allant de 50 à 200° C.

6. Procédé tel que revendiqué à la revendication 1, dans lequel l'ammonolyse est effectuée à 100° C.

7. Procédé tel que revendiqué à la revendication 1, dans lequel l'ammonolyse peut être effectuée à une pression allant de 689,475 à 3447,3 Kpa (100 à 500 psig).

8. Procédé tel que revendiqué à la revendication 1, dans lequel l'ammonolyse est effectuée à 689,475 Kpa (100 psig).

9. Procédé tel que revendiqué à la revendication 1, dans lequel des agents réducteurs sont sélectionnés à partir d'un groupe constitué d'un mélange de SnCl₂ / HCl concentré.

10. Procédé tel que revendiqué à la revendication 1, dans lequel la réduction est effectuée à une température allant de 50 à 60° C.
